(19) **Europäisches Patentamt / European Patent Office / Office européen des brevets**

(11) **EP 1 870 233 B1**

(12) **EUROPEAN PATENT SPECIFICATION**

(45) Date of publication and mention of the grant of the patent:
**09.12.2015 Bulletin 2015/50**

(51) Int Cl.:
**B32B 5/04** *(2006.01)* **B32B 27/32** *(2006.01)*

(21) Application number: **07110561.3**

(22) Date of filing: **19.06.2007**

(54) **Elastic film exhibiting low tensile force properties in the machine direction**

Elastische Folie mit niedriger Zugkraft in der Maschinenrichtung

Film élastique démontrant des propriétés de force de traction faibles dans le sens machine

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **22.06.2006 US 805569 P**

(43) Date of publication of application:
**26.12.2007 Bulletin 2007/52**

(73) Proprietor: **Tredegar Film Products Corporation Richmond, VA 23225 (US)**

(72) Inventors:
- **Quiram, Daniel Jonathan Midlothian, VA 23114 (US)**

- **Tribble, James D. Brazil, IN 47834 (US)**

(74) Representative: **Von Kreisler Selting Werner - Partnerschaft von Patentanwälten und Rechtsanwälten mbB Deichmannhaus am Dom Bahnhofsvorplatz 1 50667 Köln (DE)**

(56) References cited:
**WO-A1-01/60605**

**Description**

**FIELD OF THE INVENTION**

The present invention relates generally to elastic films, and more particularly, to elastic films exhibiting low tensile force properties in the machine direction. Such elastic films have a wide range of potential uses in both durable and disposable articles, but are particularly well suited for use in elastic waistbands, side panels and elsewhere in diapers, training pants and other products including absorbent products.

**DISCUSSION OF THE RELATED ART**

Elastomeric films are commonly used in disposable products, such as baby diapers and adult incontinent devices, in the areas of such products that are intended to conform to a wearer's body shape. The elastomeric materials of such films are typically tacky, and therefore can cause difficulties during manufacture of the film, and/or during manufacture of articles including the film, resulting from contact with machine components during manufacture. Additionally, monolayer elastomeric films pose significant blocking problems when stored on a roll.

Multilayer elastomeric films have been developed to avoid such difficulties. Such multilayer films typically include an elastic core layer sandwiched between a pair of relatively less tacky outer skin layers. Typically, the core and skin layers are coextruded. Polyolefin materials are commonly used for such skin layers. While desirable for their lack of tackiness, such polyolefin materials are considerably less elastic than the elastomeric core layer, and thus the resulting multilayer film is less elastic than the monolayer elastomeric core layer, primarily due to the inelasticity of the material of the outer skin layers.

Elasticity of the multilayer film can be enhanced by "activating" the film. One method of activation involves passing the films through intermeshing wheels, gears, etc. that mechanically stretch the film. Essentially, the activation process stretches both the inelastic skins and the elastic core, and imparts to the film elastic properties more similar to that of the elastic core. However, only the elastic core contracts to a substantial degree. As a result, an activated film typically has an irregular rippled or undulating surface, due largely to substantial contraction of the elastomeric core after the activation process, and insubstantial contraction of the relatively inelastic skin layers. The irregular surface can complicate subsequent manufacturing processes, e.g. when forming a diaper including such multilayer film. The elastic properties of the multilayer film change considerably after activation.

For certain end use applications and/or certain subsequent manufacturing processes, a conventional unactivated multilayer film may be too inelastic, and the corresponding activated film may be undesirably stretchable. In other words, for certain manufacturing applications, while the unactivated multilayer film may have a modulus of elasticity that is too high, the corresponding activated film may have a modulus of elasticity that is too low. The increased stretchable nature of the activated multilayer film has been found to further complicate certain subsequent manufacturing processes, particularly when low tensile force in the machine direction during manufacturing causes stretching of the film to an extent that interferes with the manufacturing process. In the present application, the machine direction (MD) is defined as the direction in which the film is moved through manufacturing equipment.

WO016065 describes an elastic film comprising an elastic core layer comprising highly elastic material, e.g. SIS, SEBS, and thinner skin layers arranged about it.

**SUMMARY OF THE INVENTION**

The present invention provides

(1) a multilayer elastic film, comprising:

at least a first and a second skin layer comprising at least one polymer selected from polypropylene; and an elastomeric core layer comprising a styrenic copolymer and being bonded between said first and second skin layer, wherein the first skin layer and the second layer comprise less than 6% of the total weight of the multilayer elastic film, and wherein the multilayer elastic film has a low force elastic value (LFEV) in the range of 9.8 to 118 g/cm (25 to 300 g/in), wherein the LFEV is defined as follows:

$$LFEV = (T_{300}) \times (R_1/R_2) \times (T_{300}/T_{50}),$$

wherein $T_{300}$ represents the tensile force in the machine direction in 300% strain as measured in g/cm (g/in) by ASTM D-882,

$R_1/R_2$ is calculated from a two cycle hysteresis test to 200%, wherein $R_1$ is the unload force at 100% strain divided by the load force at 100% strain for cycle 1 measured in the machine direction, and $R_2$ is the unload force at 100% strain divided by the load force at 100% strain for cycle 2 measured in the machine direction, and $T_{300}/T_{50}$ is the ratio of the tensile force at 300% strain divided by the tensile force at 50% strain measured in the machine direction in g/cm (g/in) by ASTM D-882; and

(2) a method of making the multilayer elastic film of (1) above comprising:

providing an elastomeric core layer bonded between a first layer and a second layer.

The thin multilayer film includes a relatively highly elastic, i.e., low tensile force, low modulus of elasticity, elastomeric core layer sandwiched between a pair of relatively inelastic skin layers. The thin multilayer film may be formed by coextruding onto a cast roll or by other conventional processes. The film may be non-apertured, or apertured. Apertured films may be apertured or perforated using known processes, such a vacuum forming, pin perforation or die cutting.

The elastomeric core layer preferably includes a highly elastic compound, such as a compound involving at least one or more block copolymers with a diene or hydrogenated diene midblock from the type A-B-A or A-B-A'. Preferably, the elastomeric core layer in accordance with the present invention includes elastomeric compounds that have a lower modulus of elasticity than elastomeric compounds typically used in conventional multilayer elastomeric films.

Each skin layer of the multilayer film is preferably thin relative to conventional skins of multilayer films, and preferably 10% by weight or less, or 6% by weight or less, and, in some embodiments, 5% by weight or less, of the multilayer film. The skin layers are preferably constructed of a polyolefin material.

Quantitatively, embodiments of the multilayer films in accordance with certain aspects of the present invention have a Low Force Elastic Value (LFEV) (as identified herein) within the range of 9.8-118 g/cm (25-300 g/in) (i.e., grams of force per inch of film width), and preferably in the range of 19.8-98.4 g/cm (50-250 g/in), more preferably in the range of 29.5-88.6 g/cm (75-225 g/in) and most preferably in the range of 29.5-68.9 g/cm (75-175 g/in). In contrast, conventional multilayer elastomeric films have LFEV values higher than 118 g/cm (300 g/in).

Many exemplary multilayer films in accordance with the present invention also have a tensile curve measured in the machine direction having a slope of 0.079 to 0.236 g/cm/% (0.2 to 0.6 g/in/%), from 50% strain to 300% strain. In contrast, conventional multilayer elastomeric films have been found to have tensile curves having slopes of considerably more than 0.236 g/cm/% (0.6 g/in/%) from 50% strain to 300% strain.

The method provides for selection of combinations of core and skin layers, core and skin layer materials and core and skin layer basis weights that provide the desired LFEV property or other combination including tensile force at 300% strain, average slope of the stress-strain curve between 50% and 300%, $R_1$, and $R_2$ as described. For example, a film may comprise an elastic compound having a relatively low modulus of elasticity could have relatively high basis weight skin layers or higher modulus of elasticity material in the skin layers. Another embodiment may be a film comprising an elastic compound having a relatively higher modulus of elasticity with relatively thinner skin layers or a lower modulus of elasticity material in the skin layers. Embodiments of the multilayer elastic film may comprise an LFEV value or a combination of properties including, tensile force at 300% strain, average slope of the stress-strain curve between 50% and 300%, $R_1$, and $R_2$, in the prescribed ranges. The combination of layers, layer materials and basis weights are selected as a function of one another such that the multilayer elastic film has the desired properties.

The present invention is directed to a multilayer elastic film. The multilayer elastic film has an LFEV or combination of properties including, tensile force at 300% strain, average slope of the stress-strain curve between 50% and 300%, $R_1$, and $R_2$, in the machine direction. Embodiments of the present invention include multilayer elastic films comprising a first skin layer, a second skin layer, and an elastomeric core layer bonded between the first skin layer and the skin second layer. Embodiments of the multilayer elastic film of the present invention have a low force elastic value in the range of 9.8 to 118 g/cm (25 to 300 g/in), and in certain embodiments, a low force elastic value in the range of 19.7 to 98.4 g/cm (50 to 250 g/in), or a low force elastic value in the range of 29.5 to 88.6 g/cm (75 to 225 g/in) and in certain applications, the multilayer elastic film has a low force elastic value in the range of 29.5 to 68.9 g/cm (75 to 175 g/in).

The multilayer elastic film of the present invention may comprise a first skin layer and a second skin layer of any weight percent that results in the desired properties in combination with the elastomeric core, but in certain applications the skin layers may comprise less than 10 % of the total weight of the multilayer elastic film, in certain applications wherein relatively thinner first and second layers may be desirable, the first skin layer and the second skin layer comprise less than 6 % of the total weight of the multilayer elastic film. In further embodiments, the first skin layer and second skin layer may be inelastic layers or have only a limited elasticity.

The first and second skin layers may comprise at least one polymer. The polymer may be a homopolymer or a copolymer. The polymers include polyolefins, polyethylenes, polypropylenes, or blends thereof, including single site catalyzed versions of the olefins (metallocenes). The first and second layers may also comprise an elastic polymer, such as, but not limited to, a polymer comprising at least one or more block copolymer with a diene or hydrogenated diene such as an A-B-A or A-B-A', for example styrenic copolymers with isoprene, butadiene, ethylene-propylene, or ethylene-butylene. Further, the first and second skin layers may comprise an olefinic block copolymer, elastomeric polyurethane, ethylene copolymer such as ethylene vinyl acetates, ethylene methyl acrylates, for example, ethylene/propylene copolymer elastomers, or ethylene/propylene/diene terpolymer elastomers.

The elastomeric core may comprise at least one polymer, wherein the polymers of the elastomeric core include styrenic copolymers. Styrenic copolymers include styrene/isoprene/styrene copolymers, styrene/butadiene/styrene copolymers, styrene/ethylene-propylene/styrene copolymers, or styrene/ethylene-butylene/styrene copolymers, for example. Other useful elastomeric compositions for use as a core layer, in addition to the styrenic copolymer, include olefinic block copolymers, elastomeric polyurethanes, ethylene copolymers such as ethylene vinyl acetates, ethylene methyl acrylates, ethylene/propylene copolymer elastomers or ethylene/propylene/diene terpolymer elastomers. Such polymers may be blended with each other and/or with other modifying additives.

The multilayer elastic film of the present invention may or may not be activated; and may comprise the properties described herein either before or after activation. An elastic film may be activated by intermeshing gears, for example. If the film is activated, the film may only be partially activated. A partially activated film will comprise areas with activation and areas without activation.

Additional embodiments are directed to a multilayer elastic film comprising a first skin layer, a second skin layer, and an elastomeric core layer bonded between the first layer and the second layer. In certain applications, it may be desirable for the elastic film to comprise an average slope of the stress-strain curve measured in the machine direction between 50% and 300% of less than 0.79 g/cm/% (2.0 g/in/%) or, preferably, of less than 0.236 g/cm/% (0.6 g/in/%) or within the range of 0.079 to 0.236 g/cm/% (0.2 to 0.6 g/in/%).

The multilayer elastic film may comprise an $R_1/R_2$ in the machine direction greater than 0.05 or preferably greater than 0.3 or in the range of 0.3 to 0.5; wherein $R_1$ is the unload force at 100% strain divided by the load force at 100% strain for cycle 1 and $R_2$ is the unload force at 100% strain divided by the load force at 100% strain for cycle 2. $R_1/R_2$ is a indicator of the amount of force required for a second strain relative to the first strain. In certain embodiments, $R_1$ may preferably be greater than 0.15.

The multilayer elastic film of the present invention may comprise additional additives such as processing additives. Additives such as dyes, pigments, antioxidants, antistatic agents, bonding or sealing aids, antiblocking agents, slip agents, heat stabilizers, photostabilizers, UV stabilizers, foaming agents, glass microspheres, reinforcing fibers, as well as other additives.

In further embodiments, the multilayer elastic films may comprise a first skin layer, a second skin layer, and an elastomeric core layer bonded between the first skin layer and the second skin layer, wherein the multilayer elastic film comprises a tensile force at 300% strain measured in the machine direction of less than 138 g/cm (350 g/in), an average slope of the stress-strain curve between 50% and 300% in the range of 0.079 to 0.236 g/cm/% (0.2 to 0.6 g/in/%), and an $R_1/R_2$ in the machine direction greater than 0.05.

Further, the invention is directed to a multilayer elastic film comprising a first skin layer, a second skin layer, and an elastomeric core layer bonded between the first skin layer and the second skin layer, wherein the multilayer elastic film has a negative hysteresis. A negative hysteresis is defined during a two cycle hysteresis test; the force for a given elongation (e.g. 200%) for cycle 2 is unexpectedly greater than the force for the given elongation for cycle 1. This characteristic is highly unusual for multilayer elastomeric film including skins, and is unexpected. In particular, multilayer elastomeric films including homopolymer polypropylene skins and SIS or SEBS elastomeric compounds in the core layer have been found to exhibit this characteristic.

Multilayer films having properties in the prescribed range in accordance with the present invention provide a desired degree of elasticity for end use applications, while also providing sufficient stiffness to avoid difficulties in processing and in certain manufacturing processes following manufacture of the film. All properties herein are measured in the machine direction unless otherwise indicated. This is so even without pre-activation of the multilayer film, i.e. activation of the multilayer film before manufacturing operations following manufacture of the multilayer film itself. While elimination of the need for the activation process is advantageous in certain applications, multilayer films in accordance with the present invention may also be pre-activated if desired, e.g. in a conventional manner.

## BRIEF DESCRIPTION OF THE DRAWINGS

The present invention will now be described by way of example with reference to the following drawings in which:

FIG. 1 is an enlarged cross-section of an exemplary multilayer film in accordance with the present invention; and

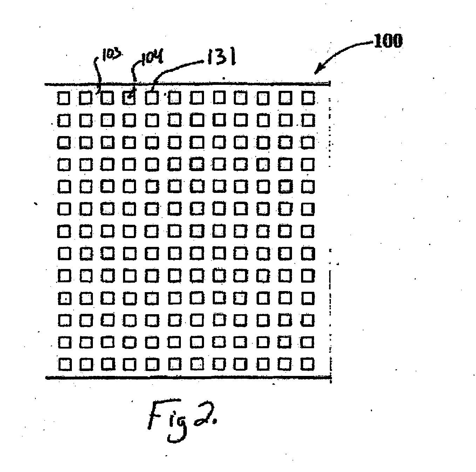

FIG. 2 is a top plan view of a textured surface of the film of FIG. 1 showing the absence of activated zones.

## DETAILED DESCRIPTION

The present invention relates to elastic films and methods of making elastic films. The elastic films have low tensile force properties in the machine direction. The low tensile forces and consistent tensile forces across the strain range of 50% to 300% allow ease of use when incorporated in disposable articles such as diapers, elastic waist bands, side panels, training pants, incontinence articles, as well as other products.

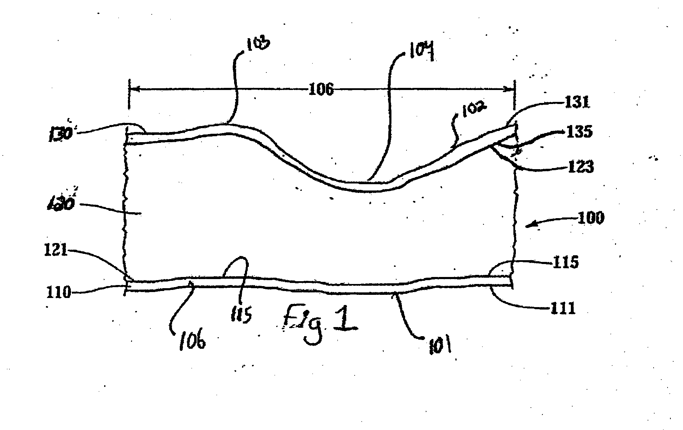

Referring now to the figures, and particularly to FIG. 1, there is shown an enlarged cross-section of an exemplary multilayer film 100 in accordance with the present invention. It has outer skin surfaces 101, 102. Optionally, outer surface 102 is a textured surface that has texture peaks 103 and texture valleys 104.

The multilayer film 100 may be formed by a conventional coextrusion process. An exemplary cast process is disclosed in U.S. Patent No. 6,472,084 to Middlesworth et al. Other extrusion and bonding processes are well known in the art. In embodiments in which the film is formed by a cast process, one or more of the surfaces may be one of flat, matte, embossed or glossy.

In one embodiment, the film has been embossed and the surface 102 is textured. In another embodiment, the film has been apertured and the surface 102 includes apertures. Embossing and aperturing of the film may be performed in a conventional embossing or vacuum forming process. U.S. Patent No. 5,733,628 to Pelkie is an example of a preferred vacuum forming process for multilayer elastic films.

Referring again to FIG. 1, the exemplary film 100 includes a first skin layer 110, a core layer 120 and a second skin layer 130. The core layer 120 has first core layer surface 121 and a second core layer surface 123. The first skin layer 110 includes a first layer inner surface 115 adjacent to the first core layer surface 121, and a first layer outer surface 111 which forms outer surface 101 of the film 100. The second skin layer 130 includes a second layer inner surface 135 adjacent to the second core layer surface 123 and a second layer outer surface 131 which forms the other (e.g. textured) surface 102 of the film 100. Preferably, the core layer 120 is in substantially continuous contact with each of the first and second layers 110, 130.

While the exemplary film is discussed for illustrative purposes as a three-layer film, it will be appreciated by those skilled in the art that multilayer films in accordance with the present invention may have more than three layers. For example, an exemplary five-layer film may include two outer skin layers, and three distinct elastomeric layers making up the central elastomeric core layer. The film may also include tie layers, as known in the art.

Accordingly, as described above, the exemplary film 100 is somewhat similar in overall layered structure to conventional films.

Conventional multilayer elastomeric films have included one or more of a relatively thick skin layers, each of which is typically from 7% to 15% by weight or more for each side of the film. Such thick skins are preferred in conventional multilayer films for various reasons, including decreased cost of the film due to the lower percentage of the expensive elastomeric core, avoidance of draw resonance during extrusion and increased tear strength of the film. The skin layers impart anti-blocking properties and enhance manufacturing processability. The unactivated skin layers impart as much or more tensile force to the film than the elastic core does.

In contrast to such conventional elastomeric multilayer films, the exemplary multilayer film comprises one or more of a relatively thin skin layer, each of which is 10% by weight or less, and preferably 6% by weight or less, or 5% by weight or less of the multilayer film. Each skin layer 110, 130 is constructed of a relatively inelastic compound, and preferably a polyolefin, such as polyethylene, polypropylene, or blends thereof, including single site catalyzed versions of the olefins (metallocenes). The skins may also contain elastic materials such as a compound involving at least one or more block copolymers with a diene or hydrogenated diene from the type A-B-A or A-B-A', such as styrene/isoprene, butadiene, ethylene-propylene, or ethylene-butylene/styrene (SIS, SBS, SEPS or SEBS) block copolymers. Other useful elastic materials include olefinic block copolymers, elastomeric polyurethanes, ethylene copolymers such as ethylene vinyl acetates, ethylene methyl acrylates, ethylene/propylene copolymer elastomers or ethylene/propylene/diene terpolymer elastomers.

In contrast to conventional multilayer elastomeric films, the present invention typically may comprise a compound in the core layer 120 that has inherently lower tensile force properties, i.e. has a relatively lower modulus of elasticity relative to conventional core materials, such as a low-modulus compound involving an SIS elastomer.

By way of further example, the core layer preferably includes a highly-elastic compound, such as a compound involving at least one or more block copolymers with a diene or hydrogenated diene from the type A-B-A or A-B-A'. Usually such a compound exhibits relatively good elastic recovery or low set from stretching over 100 percent when extruded alone as a single layer. Styrene/isoprene, butadiene, ethylene-propylene, or ethylene-butylene/styrene (SIS, SBS, SEPS or SEBS) block copolymers are particularly useful. Other useful elastomeric compositions for use as a core layer 120, in addition to the styrenic copolymer, can include olefinic block copolymers, elastomeric polyurethanes, ethylene copolymers such as ethylene vinyl acetates, ethylene methyl acrylates, ethylene/propylene copolymer elastomers or ethylene/propylene/diene terpolymer elastomers. Blends of these polymers alone or with other modifying elastic or non-elastomeric materials are also contemplated as being useful with the present invention.

It should be appreciated, however, that any suitable material may be selected, provided that the LFEV characteristic of the multilayer film falls within 9.8-118 g/cm (25-300 g/in) (i.e., grams of force per inch of film width), and preferably in the range of 19.7-98.4 g/cm (50-250 g/in), more preferably in the range of 29.5-88.6 g/cm (75-225 g/in) and most preferably in the range of 29.5-68.9 g/cm (75-175 g/in).

The LFEV quantifies certain unique properties of these MD extensible films. LFEV is defined as follows:

$$LFEV = (T_{300}) \times (R_1/R_2) \times (T_{300}/T_{50})$$

In the equation above, $T_{300}$ represents the tensile force in the machine direction at 300% strain, as measured in g/cm (g/in). At this strain level, low tensile forces are needed due to the process for stretching and converting the film into a laminate with non-woven materials. The low force properties of the film at 300% elongation contribute to the unique properties of the multilayer films described herein. The tensile properties in the machine direction may be measured using the method of ASTM D-882.

Further, $R_1/R_2$ is calculated from a two cycle hysteresis test to 200%. $R_1$ is the unload force at 100% strain divided by the load force at 100% strain for cycle 1 measured in the machine direction. $R_2$ is the unload force at 100% strain divided by the load force at 100% strain for cycle 2 measured in the machine direction. The portion of the test when the sample is pulled is referred to as the load; when the sample is allowed to relax and the grips return to their initial starting point is the unload. If a film is unactivated, $R_1$ will be relatively low compared with a film that has been previously activated. For the elastic films discussed herein, $R_1$ is generally less than $R_2$. $R_1/R_2$ will approach 1 for an activated film, and will be less than 1 for an unactivated film. A suitable procedure for measuring hysteresis of a sample is described in U.S. Patent No. 6,472,084 at column 8, lines 9-33, and is performed to 200% strain consistent therewith.

$T_{300}/T_{50}$ as used herein is a ratio of tensile force values measured in the machine direction (tensile force at 300% divided by tensile force at 50%) and it relates to how flat the tensile curve is from 50% to 300% strain. If the data are flat in this region, this value will approach 1; if the data are sloped in this region, this value can be much greater than 1. The test method of ASTM D-882 may be used to determine $T_{300}/T_{50}$.

Exemplary elastomeric multilayer films in accordance with the present invention have an LFEV in the range of 9.8-118 g/cm (25-300 g/in), and preferably in the range of 19.7-98.4 g/cm (50-250 g/in), in the range of 29.5-88.6 g/cm (75-225 g/in), and in the range of 29.5-68.9 g/cm (75-175 g/in).

Exemplary films have been produced by combining low force elastics in the core with thin polyolefin skins (less than 6% by weight per side). These films may be unactivated and have an initial tensile force value of less than 138 g/cm (350g/inch) at 300% elongation in the machine direction. In addition to the low force tensile properties of these films in the machine direction, they also have a reasonably flat tensile curve in the range of 50% through 300% elongation. See Tensile Slope values in Table 1 below.

Exemplary embodiments provide a multilayer film having relatively low tensile force, i.e. a relatively low modulus of elasticity, even before any activation process for the multilayer film. Accordingly, an unactivated multilayer film according to the present invention provides a desirable balance between stiffness in the machine direction, thus enhancing manufacturing processability, and elasticity of the multilayer film as a whole.

Data relating to exemplary multilayer elastomeric films in accordance with the present invention are shown in Table 1 below.

**TABLE 1**

| Property | Units | Film 1* | Film 2* | Film 3* | Film 4* | Film 5 | Film 6* | Film 7* | Film 8* | Film 9* | Film 10* |
|---|---|---|---|---|---|---|---|---|---|---|---|
| Skins | | PE | PE | PE | PE w/ Sealing Additive | PP | PE | PE | PE | PE | PE |
| Core | | SIS | SIS | SIS | SIS | SIS | SIS | SIS | SIS | SIS | SIS |
| Processing Method | | Vac Form | VF | VF | VF | VF | Cast | VF | VF | VF | VF |
| Basis Weight | gsm | 29 | 29 | 30 | 30 | 31 | 32 | 27 | 31 | 20 | 30 |
| Coefficient of Friction | | 2.3 | 2.1 | 1.9 | 2.0 | 0.8 | | | | | |
| MD Tear | g | 253 | 399 | 252 | 381 | 424 | 297 | 229 | | 148 | 237 |
| TD Tear | g | 293 | 333 | 246 | 192 | 472 | 461 | 295 | | 176 | 218 |
| Air Permeability | cfm | | | | | | | | | | 165 |
| Tensile Slope - 50% to 300% | g/cm/% (g/in/%) | 0.17 (0.43) | 0.14 (0.36) | 0.11 (0.29) | 0.18 (0.46) | 0.079 (0.20) | 0.18 (0.46) | 0.18 (0.46) | 0.20 (0.52) | 0.13 (0.32) | 0.19 (0.49) |
| R1 | | 0.197 | 0.221 | 0.297 | 0.198 | 0.187 | 0.280 | 0.199 | 0.174 | 0.192 | 0.175 |
| R2 | | 0.587 | 0.521 | 0.592 | 0.636 | 0.567 | 0.649 | 0.600 | 0.470 | 0.532 | 0.547 |
| R1/R2 | | 0.335 | 0.424 | 0.502 | 0.311 | 0.330 | 0.431 | 0.331 | 0.371 | 0.361 | 0.320 |
| Hysteresis | % | 10 | 6 | 2 | 12 | -3 | 7 | | 9 | 8 | 8 |
| MD Tensile @ 300% (T300) | g/cm (g/in) | 102 (259) | 83 (212) | 73 (185) | 110 (279) | 87 (220) | 119 (302) | 107 (272) | 115 (291) | 71 (181) | 98 (250) |
| T300/T50 | | 1.715 | 1.738 | 1.652 | 1.701 | 1.294 | 1.624 | 1.722 | 1.796 | 1.792 | 1.953 |
| Low Force Elastic Value (LFEV) | g/cm (g/in) | 59 (149) | 61 (156) | 60 (153) | 58 (148) | 37 (94) | 83 (211) | 61 (155) | 76 (194) | 46 (117) | 61 (156) |

*Reference Example

EP 1 870 233 B1

In Table 1 above, Air Permeability is determined by testing in accordance with ASTM D737. Also in Table 1 above, "VF" signifies vacuum formed film vs. a film produced via a cast process. The Machine Direction ("MD") and Transverse Direction ("TD") tear values are determined by an Elmendorf tear test (ASTM D1922), and Coefficient of Friction values between film and steel are determined by testing according to ASTM D1894.

In a preferred embodiment, the film 100 includes only non-activated zones 106, and no portion of the film is activated, yet the film has the desired LFEV. Alternatively, the film includes activated zones and has the desired LFEV.

To provide a film in the LFEV range set forth, a combination of core resin(s), skins resin(s), and basis weights must be chosen. A variety of core materials, skin materials, and basis weights provide multilayer films having an LFEV within the prescribed range, for each desired combination of core and skin layers. These factors are interrelated and must be selected in combination to provide a multilayer film having an LFEV in the prescribed range.

In accordance with an exemplary method in accordance with the present invention, the desired combination of core and skin layers is first determined. For example, consider that it is desired to have a single core layer between a pair of skin layers.

Next, the amount of force that the elastomeric core provides per unit film measure is determined as a function of selection of elastomeric material. In the exemplary embodiment, an elastomeric compound for the core was chosen that provides tensile force in the range of 1.38-1.54 g/cm/gsm (3.5 - 3.9 g/in/gsm) tensile force at 300% extension in the machine direction ($T_{300}$). This value is relatively low for elastomeric compounds. Other common film grade elastomeric compounds that provide greater forces can also be used, as discussed below.

Next, a core layer basis weight is selected. The basis weight is selected in view of the tensile force properties of the elastomeric compound selected. For an exemplary embodiment (see Table 1, Film 5) exhibiting 1.38-1.54 g/cm/gsm (3.5-3.9 g/in/gsm) tensile force, selection of a 27.6 gsm core layer basis weight will result in the core layer providing tensile force in the range of 38-43 g/cm (97-108 g/in).

Accordingly, since the selected core layer will provide relatively low tensile force, skin layer materials (and/or basis weights) may be selected that provide up to a relatively higher tensile force, since these factors are interrelated.

Next, skin layer materials are selected, in view of the tensile force that will be provided by the core layer. For the exemplary embodiment, polyolefin skins (polyethylene and polypropylene) were selected that contribute 16-24 g/cm/gsm (40-60 g/in/gsm) towards $T_{300}$.

Next skin layer basis weights are selected, in view of the tensile force that will be provided by the core layer and the materials selected for the skin layers. For an exemplary embodiment having 2.4 gsm (1.2 gsm basis weight per side), the skin layers will provide 38-57 g/cm (96-144 g/in) towards $T_{300}$.

Accordingly a multilayer film comprising this elastomeric core layer and these skins layers will have a $T_{300}$ value of 76-99 g/cm (193-252 g/in) (note Film 5 in Table 1 at 87 g/cm (220 g/in)), which is well below the value of 138 g/cm (350 g/in) specified.

For an unactivated film, $R_1/R_2$ will be relatively low (0.2-0.6). Therefore, for a $T_{300}$ value of 220 g/in (note Film 5 in Tables 1), an $R_1/R_2$ value of 0.33 and a $T_{300}/T_{50}$ value of 1.29, this exemplary multilayer film has an LFEV of 94 g/in, which is well within the specified LFEV range.

It will be appreciated by those skilled in the art that these parameters may be determined in any order, and that in the event a first selection yields an LFEV outside of the range, one or more of the parameters discussed above may be reselected to provide for a combination of layers, layer materials and basis weights that provide a multilayer film having an LFEV within the prescribed range.

While the exemplary embodiment above includes an elastomer having a tensile force in the range of 1.4-1.5 g/cm/gsm (3.5-3.9 g/in/gsm) towards $T_{300}$, suitable formulations may also be determined for other elastomeric compounds. By way of alternative example, for a selected elastomeric compound providing 2.9 g/cm/gsm (7.4 g/in/gsm) to $T_{300}$, and a selected 27.6 gsm core layer basis weight, the core layer will provide 80 g/cm (204 g/in) of tensile force towards $T_{300}$. For a polyolefin skin layer basis weight of 2.4 gsm (1.2 gsm/side), the skin layers will provide 38-57 g/cm (96-144 g/in) force towards $T_{300}$. Accordingly, $T_{300}$ is 300-348 g/in for a multilayer film including the core and skin layers. Thus, this alternative embodiment of a multilayer film in accordance with the present invention includes a relatively higher force/high modulus elastomer (approximately twice the load of the exemplary embodiment above), and still falls within the force specification range.

For a $T_{300}$ value of 137 g/cm (348 g/in), an $R_1/R_2$ value of 0.4 and a $T_{300}/T_{50}$ value of 1.65, LFEV is 91 g/cm (230 g/in), which is still well within the specified LFEV range. Thus, by way of example, an elastomeric core providing twice the force of the exemplary embodiment may be utilized to make a film within the preferred LFEV range.

Various other combinations are feasible. In general, a film including an elastic compound having a relatively low modulus of elasticity may have relatively thick (or higher modulus of elasticity material in the) skin layers, and a film including an elastic compound having a relatively higher modulus of elasticity may require relatively thinner (or lower modulus of elasticity material in the) skin layers. The number of layers, layer materials and basis weights are selected as a function of one another such that a multilayer film including the selected layers, layer materials and basis weights has the desired LFEV property.

It is recognized that higher basis weight films and films with higher basis weight skins are easier to process. As a result, there are advantages to selecting an elastomeric compound for the core layer that provides a low force (i.e. a low modulus of elasticity).

A certain exemplary embodiment of the multilayer film includes an SIS triblock copolymer core and polypropylene skins and a 4/92/4 skin/core/skin percentage by weight of the firm. Embodiments having polypropylene skins (see data for Film 5 in Table 1above), and an SIS or SEBS elastomeric compound in the core layer, have been found to have a unique and unexpected characteristic during hysteresis testing. Specifically, such polypropylene skinned embodiments have been found to exhibit negative hysteresis, meaning, for example, that during a two cycle hysteresis test to 200% elongation, the force at 200% for cycle 2 is unexpectedly greater than the force at 200% for cycle 1. This characteristic is highly unusual for elastomeric films with skins, and is unexpected. Negative hysteresis data for exemplary films is illustrated in Table 1 above. An exemplary Hysteresis Graph is shown below; note the plot for Film 5, which corresponds to a multilayer elastomeric film having thin polypropylene skins in accordance with the present invention.

Hysteresis Plot

An exemplary film has been made using the conventional vacuum formed (VF) process referred to above, using relatively low vacuum pressure to produce a deeply embossed film. Due to the low forces needed to stretch this film in the MD direction, this deep embossment provides several processing advantages. These advantages include a film that is less likely to block, can be unrolled with much smaller forces, and has a lesser tendency to wrinkle. Due to these advantages, the deeply embossed films are expected to be easier to process on diaper/training pant lines.

An exemplary film includes an SIS triblock copolymer core and homopolymer polypropylene skins. Embodiments including such polypropylene skins are unique relative to versions containing polyethylene-based skins because the tensile forces increase more quickly at low strains and reach a force plateau (i.e. the polypropylene makes the film stiffer at low strains). The version with polypropylene skins starts to plateau at approximately 15% elongation versus a value of approximately 50% for the polyethylene-based versions. More specifically, the slope of the tensile curve from 50% strain to 300% strain is close to 0. For example, a slope of the tensile curve of 0.079 to 0.236 g/cm/% (0.2 to 0.6, g/in/%) from 50% strain to 300% strain is close to 0. An exemplary tensile graph is shown below; note the plateau (slope close to 0) for Film 5, which corresponds to a multilayer elastomeric film in accordance with the present invention. The difference in shape of the tensile curves from 0-50% elongation is quite distinctive. Because of these unique tensile properties, the PP skins are at the low end of the LFEV of interest.

**Tensile Graph**

The exemplary film with polypropylene skins has further differences and advantages. For example, it has a lower coefficient of friction (COF) than the polyethylene-based skin versions; this may offer processing advantages on a diaper line. The lower COF may allow the film to slide over some of the rolls easier, avoiding localized high stress concentrations in the film. Another advantage is strength of hot melt adhesion to the film. For hygiene applications, this film can be stretched and non wovens are laminated to both sides using a hot melt adhesive. Hot melt adhesives have been found to adhere significantly better to the film with polypropylene skins (higher bond strengths). Another advantage is strength of adhesion when ultrasonic bonding is used. For hygiene applications, this film can be stretched and non-wovens are laminated to both sides using ultrasonic bonding.

Numerous embodiments of the present invention have been prepared and are shown in Table 2. The multilayer films of Table 2 typically include a relatively highly elastic, i.e., low tensile force, low modulus of elasticity, elastomeric core layer sandwiched between a pair of relatively inelastic skin layers. The films may be non-apertured, or apertured. Apertured films may be apertured or perforated using known processes, such a vacuum forming, pin perforation or die cutting. Embodiments ID# 22, 23, 50, 62, and 63 comprised apertures and the properties presented are for the apertured film. As can be seen, there are multiple combinations of core material, skin material, core weight %, skin weight percent, and overall basis weight that result in films having the properties of the present invention. Unexpectedly, several embodiments also include a negative hysteresis. The surprising properties of embodiments of the multilayer film of the present invention include exhibiting low tensile force properties in the machine direction.

Most of the skin layers of the multilayer elastic films of Table 2 are relatively thin. The skin layer may be any thickness that results in the desired properties based upon core properties and skin properties, as shown in embodiments in Table 2, the skin layers may be a higher weight percent but preferably each skin layer is 10% by weight or less, or preferably, 6% by weight or less. The skin layers are preferably constructed of a polyolefin material.

Quantitatively, embodiments of the multilayer films in Table 2 have properties in accordance with the present invention including having an LFEV within the broad range of 9.8-118 g/cm (25-300 g/in), having a tensile curve measured in the machine direction having a slope of nearly 0, preferably less than 0.236 g/cm/% (0.6 g/in/%) or from 0.079 to 0.236 g/cm/% (0.2 to 0.6 g/in/%), from 50% strain to 300% strain, having a tensile force at 300% strain of less than 350 g/in, $R_1/R_2$ in the machine direction in the range of 0.3 to 0.5, or combinations of these properties. The combination of layers, layer materials and basis weights are selected as a function of one another such that a multilayer film including the selected layers, layer materials and basis weights has the desired properties. The unique properties of the films of Table 2 allow the elastic film to elongate easily and with a consistent tensile force throughout the range of 50% to 300% elongation.

Multilayer films having properties in the prescribed range in accordance with the present invention provide a desired degree of elasticity for end use applications, while also providing sufficient stiffness to avoid difficulties in certain manufacturing processes following manufacture of the film. All properties herein are measured in the machine direction unless otherwise indicated. This is so even without pre-activation of the multilayer film, i.e. activation of the multilayer film before manufacturing operations following manufacture of the multilayer film itself. While elimination of the need for the activation process is advantageous in certain applications, multilayer films in accordance with the present invention may also be pre-activated if desired, e.g. in a conventional manner.

Table 2

| ID # | Core Comp. | Skin Comp. | Basis wt., Gsm | Skin 1 wt. % | Skin 2 wt.% | Total Skin, wt.% | Hysteresis, % | $R_1$ | $R_2$ | $R_1/R_2$ | $T_{300}$, gf/cm (gf/in) | $T_{300}/T_{50}$ | LFEV, gf/cm (gf/in) | Avg. Slope (50%- 300%), g/cm/% (g/in/%) |
|---|---|---|---|---|---|---|---|---|---|---|---|---|---|---|
| 11* | SIS | PE | 29.5 | | | | 10 | 0.194 | 0.581 | 0.334 | 102 (259) | 1.711 | 58 (148) | 0.17 (0.43) |
| 12* | SIS | PE w/Sealing Additive | 29.4 | | | | 6 | 0.221 | 0.515 | 0.428 | 83 (212) | 1.742 | 62 (158) | 0.14 (0.36) |
| 13* | SIS | PE | 29.5 | | | | 2 | 0.299 | 0.593 | 0.504 | 73 (185) | 1.653 | 61 (154) | 0.11 (0.29) |
| 14* | SIS | PE | 29.5 | | | | 12 | 0.197 | 0.639 | 0.307 | 110 (279) | 1.694 | 53 (145) | 0.18 (0.46) |
| 15 | SIS | PP | 31.1 | 5.0 | 3.5 | 8.5 | -3 | 0.188 | 0.559 | 0.336 | 87 (220) | 1.298 | 38 (96) | 0.08 (0.20) |
| 16* | SIS | PE | 30.0 | | | | 8 | 0.225 | 0.632 | 0.356 | 105 (267) | 1.549 | 58 (147) | 0.15 (0.38) |
| 17* | SIS | PE | 32.3 | 3.4 | 3.3 | 6.7 | 7 | 0.282 | 0.646 | 0.437 | 119 (302) | 1.618 | 84 (213) | 0.18 (0.46) |
| 18* | SIS | PE | 27.3 | | | | 10 | 0.198 | 0.607 | 0.326 | 107 (272) | 1.720 | 60 (153) | 0.18 (0.46) |
| 19* | SIS | PE | 31.1 | | | | 8 | 0.174 | 0.474 | 0.367 | 115 (291) | 1.791 | 75 (191) | 0.20 (0.51) |
| 20* | SIS | PE | 30.4 | | | | 9 | 0.178 | 0.480 | 0.372 | 111 (283) | 1.798 | 74 (189) | 0.20 (0.50) |
| 21* | SIS | PE | 19.7 | | | | 8 | 0.195 | 0.534 | 0.366 | 71 (181) | 1.789 | 47 (119) | 0.13 (0.32) |
| 22* | SIS | PE | 27.9 | | | | 10 | 0.157 | 0.546 | 0.288 | 104 (264) | 1.970 | 59 (149) | 0.20 (0.52) |
| 23* | SIS | PE | 30.2 | | | | 8 | 0.173 | 0.553 | 0.313 | 98 (250) | 1.951 | 60 (152) | 0.19 (0.49) |
| 24* | SIS | PE | 30 | | | | 7 | 0.190 | 0.518 | 0.367 | 110 (281) | 1.791 | 73 (185) | 0.20 (0.50) |
| 25* | SIS | PE | 30 | | | | 8 | 0.197 | 0.543 | 0.363 | 116 (294) | 1.771 | 74 (189) | 0.20 (0.51) |
| 26* | SIS | PE w/Sealing Additives | 30 | | | | 4 | 0.261 | 0.521 | 0.501 | 78 (198) | 1.651 | 65 (164) | 0.12 (0.31) |
| 27* | SIS | PE w/Sealing Additives | 30 | | | | 6 | 0.229 | 0.479 | 0.478 | 94 (239) | 1.681 | 76 (192) | 0.15 (0.39) |
| 28* | SIS | PE | 30 | | | | 4 | 0.269 | 0.511 | 0.526 | 84 (214) | 1.596 | 71 (180) | 0.13 (0.32) |
| 29 | SIS | PP | 29.6 | 6.0 | 4.3 | 10.3 | -3 | 0.135 | 0.495 | 0.272 | 107 (271) | 1.219 | 35 (90) | 0.08 (0.20) |
| 30 | SIS | PP | 27.8 | 6.8 | 4.6 | 11.3 | -2 | 0.125 | 0:470 | 0.267 | 99 (252) | 1.249 | 33 (84) | 0.08 (0.20) |
| 31 | SIS | PP | 29.5 | 4.9 | 4.1 | 9.0 | 1 | 0.152 | 0.537 | 0.284 | 97 (246) | 1.245 | 34 (87) | 0.07 (0.19) |

(continued)

| ID # | Core Comp. | Skin Comp. | Basis wt, Gsm | Skin 1 wt. % | Skin 2 wt. % | Total Skin, wt.% | Hysteresis, % | $R_1$ | $R_2$ | $R_1/R_2$ | $T_{300}$, gf/cm (gf/in) | $T_{300}/T_{50}$ | LFEV, gf/cm (gf/in) | Avg. Slope (50%-300%), g/cm/% (g/in/%) |
|---|---|---|---|---|---|---|---|---|---|---|---|---|---|---|
| 32 | SIS | PP | 30.0 | 4.4 | 2.8 | 7.2 | -2 | 0.219 | 0.600 | 0.365 | 93 (236) | 1.412 | 48 (122) | 0.11 (0.28) |
| 33 | SIS | PP | 30.3 | 4.2 | 3.4 | 7.6 | -2 | 0.185 | 0.560 | 0.330 | 96 (245) | 1.356 | 43 (110) | 0.10 (0.26) |
| 34 | SIS | PP | 29.7 | 4.4 | 3.3 | 7.7 | -1 | 0.198 | 0.579 | 0.341 | 90 (229) | 1.408 | 43 (110) | 0.11 (0.27) |
| 35 | SIS | PP | 28.7 | 4.3 | 3.4 | 7.7 | -1 | 0.181 | 0.556 | 0.325 | 93 (235) | 1.408 | 42 (107) | 0.11 (0.27) |
| 36 | SIS | PP | 30.2 | 5.8 | 4.4 | 10.2 | -2 | 0.134 | 0.489 | 0.274 | 117 (297) | 1.327 | 43 (108) | 0.11 (0.29) |
| 37 | SIS | PP | 35.6 | 6.1 | 4.6 | 10.6 | -1 | 0.120 | 0.476 | 0.252 | 134 (341) | 1.237 | 42 (107) | 0.10 (0.26) |
| 38* | SIS | PP | 25.5 | 9.0 | 7.3 | 16.3 | 1 | 0.041 | 0.270 | 0.153 | 122 (311) | 1.273 | 24 (61) | 0.10 (0.27) |
| 39 | SIS | PP | 27.6 | 4.0 | 2.9 | 6.9 | -1 | 0.179 | 0.541 | 0.330 | 85 (216) | 1.438 | 41 (103) | 0.10 (0.26) |
| 40 | SIS | PP | 24.9 | 4.6 | 2.5 | 7.1 | -1 | 0.210 | 0.570 | 0.368 | 80 (202) | 1.430 | 42 (106) | 0.09 (0.24) |
| 41 | SIS | PP | 35.3 | 3.5 | 2.5 | 6.0 | -1 | 0.218 | 0.582 | 0.374 | 104 (263) | 1.418 | 55 (140) | 0.12 (0.31) |
| 42 | SIS/SEBS Blend | PP | 30.3 | 4.5 | 3.8 | 8.3 | 2 | 0.167 | 0.503 | 0.332 | 126 (321) | 1.466 | 61 (156) | 0.16 (0.41) |
| 43 | SIS/SEBS Bend | PP | 24.9 | 4.2 | 3.0 | 7.2 | 3 | 0.210 | 0.564 | 0.372 | 98 (249) | 1.528 | 56 (142) | 0.13 (0.34) |
| 44 | SIS/SEBS Blend | PP | 30.2 | 4.8 | 3.5 | 8.2 | 7 | 0.215 | 0.530 | 0.405 | 144 (365) | 1.602 | 93 (237) | 0.22 (0.55) |
| 45 | SIS/SEBS Blend | PP | 31.2 | 3.9 | 2.5 | 6.5 | 0 | 0.220 | 0.598 | 0.368 | 97 (247) | 1.416 | 50 (128) | 0.11 (0.29) |
| 46 | SEBS | PP | 30.2 | 6.1 | 5.0 | 11.1 | -2 | 0.133 | 0.500 | 0.266 | 105 (267) | 1.276 | 36 (91) | 0.09 (0.23) |
| 47 | SIS | PP | 31.8 | 4.8 | 3.7 | 8.4 | 1 | 0.138 | 0.513 | 0.270 | 119 (303) | 1.280 | 41 (105) | 0.11 (0.27) |
| 48 | SIS | PP | 30.4 | 3.4 | 3.4 | 6.8 | -1 | 0.210 | 0.574 | 0.367 | 86 (218) | 1.412 | 44 (113) | 0.10 (0.25) |
| 49 | SIS | PP | 30.9 | 4.8 | 4.6 | 9.5 | -2 | 0.121 | 0.466 | 0.259 | 117 (289) | 1.273 | 37 (95) | 0.10 (0.25) |
| 50 | SIS | PP | 29.1 | 3.0 | 2.9 | 5.9 | 0 | 0.245 | 0.594 | 0.413 | 78 (198) | 1.453 | 47 (119) | 0.10 (0.25) |
| 51 | SIS w/Antiblock | PP w/Antiblock | 30.9 | 3.2 | 3.2 | 6.5 | 0 | 0.226 | 0.587 | 0.385 | 97 (246) | 1.493 | 56 (141) | 0.13 (0.32) |
| 52 | SIS | PP | 29.4 | 4.4 | 3.9 | 8.3 | -1 | 0.172 | 0.538 | 0.319 | 98 (248) | 1.367 | 43 (108) | 0.11 (0.27) |
| 53* | SIS/PE Blend | PE | 40.3 | 6.5 | 5.9 | 12.4 | 11 | 0.033 | 0.171 | 0.192 | 339 (862) | 1.551 | 101 (256) | 0.48 (1.22) |
| 54* | SIS/PE Blend | PE | 30.7 | 6.8 | 6.0 | 12.8 | 11 | 0.020 | 0.148 | 0.133 | 282 (717) | 1.571 | 59 (150) | 0.40 (1.04) |

(continued)

| ID # | Core Comp. | Skin Comp. | Basis wt, Gsm | Skin 1 wt. % | Skin 2 wt. % | Total Skin, wt.% | Hysteresis, % | $R_1$ | $R_2$ | $R_1/R_2$ | $T_{300}$, gf/cm (gf/in) | $T_{300}/T_{50}$ | LFEV, gf/cm (gf/in) | Avg. Slope (50%-300%), g/cm/% (g/in/%) |
|---|---|---|---|---|---|---|---|---|---|---|---|---|---|---|
| 55 | SIS/PE Blend | PP | 40.0 | 5.9 | 5.7 | 11.6 | 8 | 0.014 | 0.085 | 0.159 | 347 (882) | 1.328 | 73 (186) | 0.34 (0.87) |
| 56 | SIS/PE Blend | PP | 30.8 | 5.8 | 5.5 | 11.3 | 9 | 0.007 | 0.050 | 0.134 | 296 (753) | 1.390 | 56 (141) | 0.33 (0.85) |
| 57* | SIS | PE | 40.9 | 12.7 | 11.2 | 23.9 | 6 | 0.012 | 0.196 | 0.063 | 258 (656) | 1.415 | 23 (59) | 0.30 (0.77) |
| 58* | SIS | PE | 31.7 | 12.6 | 11.1 | 23.7 | 7 | 0.014 | 0.207 | 0.067 | 224 (570) | 1.425 | 21 (54) | 0.27 (0.68) |
| 59 | SIS | PP | 30.8 | | | | 0 | 0.124 | 0.477 | 0.259 | 99 (252) | 1.235 | 32 (81) | 0.07 (0.19) |
| 60 | SIS | PP | 31.0 | | | | 1 | 0.117 | 0.468 | 0.251 | 102 (258) | 1.206 | 31 (78) | 0.07 (0.18) |
| 61 | SIS | PP | 29.8 | | | | -1 | 0.108 | 0.450 | 0.240 | 102 (259) | 1.248 | 31 (78) | 0.08 (0.21) |
| 62 | SIS | PP | 29.7 | | | | -2 | 0.107 | 0.413 | 0.260 | 89 (225) | 1.515 | 35 (89) | 0.12 (0.31) |
| 63 | SIS | PP | 29.4 | | | | -3 | 0.101 | 0.400 | 0.253 | 94 (240) | 1.509 | 36 (92) | 0.13 (0.32) |
| 64 | SEBS | PP | 30.7 | | | | -2 | 0.093 | 0.420 | 0.220 | 124 (314) | 1.389 | 38 (96) | 0.14 (0.35) |
| 65 | SEBS | PP | 30.8 | | | | -3 | 0.094 | 0.419 | 0.223 | 126 (320) | 1.346 | 38 (96) | 0.13 (0.33) |
| 66 | SEBS | PP | 29.9 | | | | -2 | 0.104 | 0.452 | 0.230 | 115 (291) | 1.345 | 35 (90) | 012 (0.30) |
| 67 | SIS | PP | 29.6 | 4.4 | 3.5 | 8.0 | -3 | 0.183 | 0.550 | 0.333 | 102 (258) | 1.487 | 50 (128) | 0.13 (0.34) |
| 68 | SIS | PP | 24.0 | 4.0 | 3.0 | 7.0 | -3 | 0.210 | 0.572 | 0.367 | 80 (204) | 1.591 | 47 (119) | 0.12 (0.30) |
| 69* | SIS | PP | 21.1 | 6.9 | 5.6 | 12.5 | -5 | 0.086 | 0.374 | 0.229 | 93 (237) | 1.458 | 31 (79) | 0.12 (0.30) |
| 70 | SIS w/Sealing Additive | PP | 33.2 | 2.8 | 2.7 | 5.5 | 7 | 0.124 | 0.424 | 0.293 | 144 (366) | 1.743 | 74 (187) | 0.24 (0.62) |
| 71 | SIS w/Sealing Additive | PP | 25.3 | 3.4 | 2.9 | 6.3 | 8 | 0.097 | 0.395 | 0.245 | 134 (340) | 1.689 | 56 (141) | 0.22 (0.55) |
| 72 | SIS w/Sealing Additive | PP | 20.8 | 3.3 | 3.0 | 6.3 | 8 | 0.105 | 0.431 | 0.244 | 117 (297) | 1.723 | 49 (125) | 0.20 (0.50) |
| 73 | SEBS | PP | 26.7 | 4.1 | 3.2 | 7.3 | 0 | 0.169 | 0.532 | 0.318 | 92 (234) | 1.538 | 45 (114) | 0.13 (0.33) |
| 74 | SEBS | PP | 30.9 | 4.1 | 3.2 | 7.4 | 0 | 0.150 | 0.502 | 0.299 | 101 (257) | 1.584 | 48 (122) | 0.15 (0.38) |
| 75 | SEBS w/Sealing Additive | PP | 29.8 | 3.8 | 3.3 | 7.0 | 3 | 0.145 | 0.466 | 0.311 | 125 (398) | 1.649 | 64 (163) | 0.20 (0.50) |
| 76 | SEBS | PP | 30.9 | 4.4 | 3.4 | 7.7 | 3 | 0.168 | 0.514 | 0.326 | 114 (290) | 1.543 | 57 (146) | 0.16 (0.41) |

(continued)

| ID # | Core Comp. | Skin Comp. | Basis wt., Gsm | Skin 1 wt. % | Skin 2 wt.% | Total Skin, wt.% | Hysteresis, % | $R_1$ | $R_2$ | $R_1/R_2$ | $T_{300}$, gf/cm (gf/in) | $T_{300}/T_{50}$ | LFEV, gf/cm (gf/in) | Avg. Slope (50%-300%), g/cm/% (g/in/%) |
|---|---|---|---|---|---|---|---|---|---|---|---|---|---|---|
| 77* | SEBS | PP | 28.8 | 9.5 | 8.4 | 17.9 | 1 | 0.037 | 0.268 | 0.139 | 149 (378) | 1.366 | 28 (72) | 0.16 (0.40) |
| 78 | SEBS | PP | 29.7 | | | | -5 | 0.185 | 0.560 | 0.330 | 106 (268) | 1.742 | 61 (154) | 0.18 (0.46) |
| 79 | SEBS w/Sealing Additive | PP | 28.1 | | | | -1 | 0.106 | 0.410 | 0.260 | 134 (340) | 1.832 | 64 (162) | 0.24 (0.62) |
| 80 | SIS w/Sealing Additive | PP | 28.3 | | | | 1 | 0.136 | 0.445 | 0.306 | 113 (288) | 1.814 | 63 (160) | 0.20 (0.52) |
| 81 | SIS | PP | 30.3 | | | | -4 | 0.253 | 0.617 | 0.410 | 83 (211) | 1.590 | 54 (138) | 0.12 (0.31) |
| 82 | SIS | PP | 21.1 | | | | -5 | 0.112 | 0.430 | 0.262 | 90 (229) | 1.603 | 38 (96) | 0.13 (0.34) |
| 83 | SIS | PP | 29.5 | 3.5 | 2.3 | 5.7 | -3 | 0.201 | 0.557 | 0.360 | 101 (256) | 1.624 | 59 (150) | 0.15 (0.39) |
| 84 | SIS | PP/EP Copolymer Blend | 28.5 | 4.4 | 3.7 | 8.2 | -3 | 0.239 | 0.548 | 0.436 | 92 (233) | 1.578 | 63 (161) | 0.13 (0.34) |
| 85* | SIS | EP Copolymer | 28.4 | | | | -1 | 0.264 | 0.463 | 0.569 | 78 (198) | 1.682 | 75 (190) | 0.13 (0.32) |
| 86 | SIS | PP | 30.2 | 3.8 | 8.0 | 11.9 | -5 | 0.095 | 0.404 | 0.234 | 120 (306) | 1.439 | 41 (103) | 0.15 (0.37) |
| 87 | SEBS/Thermoplas tic Olefin Elastomer Blend | PP | 29.2 | | | | -1 | 0.215 | 0.445 | 0.483 | 106 (268) | 1.506 | 77 (195) | 0.14 (0.36) |
| 88 | SIS | PP | 29.8 | | | | -3 | 0.190 | 0.557 | 0.342 | 93 (237) | 1.409 | 45 (114) | 0.11 (0.27) |
| 89 | SIS | PP | 30.4 | | | | -1 | 0.207 | 0.585 | 0.354 | 86 (219) | 1.379 | 42 (107) | 0.09 (0.24) |
| 90 | SIS | PP | 29.4 | | | | 9 | 0.023 | 0.133 | 0.175 | 240 (609) | 1.371 | 57 (146) | 0.26 (0.66) |
| 91 | SEBS/Thermo plastic Olefin Elastomer Blend | PP | 31.2 | | | | 3 | 0.224 | 0.441 | 0.507 | 115 (293) | 1.383 | 81 (205) | 0.13(0.32) |
| *Reference Example | | | | | | | | | | | | | | |

EP 1 870 233 B1

While the present invention has been described primarily in the context of absorbent products and the like, it is recognized that the present invention may also be applied to many other applications and environments. For example, the present invention is particularly well-suited for use with disposable articles using the film of the present invention as a waistband component or side panel component.

**Claims**

1.  A multilayer elastic film, comprising:

    at least a first and a second skin layer comprising at least one polymer selected from polypropylene; and
    an elastomeric core layer comprising a styrenic copolymer and being bonded between said first and second skin layer, wherein the first skin layer and the second layer comprise less than 6% of the total weight of the multilayer elastic film, and wherein the multilayer elastic film has a low force elastic value (LFEV) in the range of 9.8 to 118 g/cm (25 to 300 g/in), wherein the LFEV is defined as follows:

$$LFEV = (T_{300}) \times (R_1/R_2) \times (T_{300}/T_{50}),$$

    wherein $T_{300}$ represents the tensile force in the machine direction in 300% strain as measured in g/cm (g/in) by ASTM D-882,
    $R_1/R_2$ is calculated from a two cycle hysteresis test to 200%, wherein $R^1$ is the unload force at 100% strain divided by the load force at 100% strain for cycle 1 measured in the machine direction, and $R_2$ is the unload force at 100% strain divided by the load force at 100% strain for cycle 2 measured in the machine direction, and $T_{300}/T_{50}$ is the ratio of the tensile force at 300% strain divided by the tensile force at 50% strain measured in the machine direction in g/cm (g/in) by ASTM D-882.

2.  The multilayer elastic film of claim 1, wherein the multilayer elastic film has an LFEV in the range of 19.7 to 98.4 g/cm (50 to 250 g/in), preferably in the range of 29.5 to 88.6 g/cm (75 to 225 g/in), most preferably in the range of 29.5 to 68.9 g/cm (75 to 175 g/in).

3.  The multilayer elastic film of claim 1, wherein the multilayer elastic film is not activated.

4.  The multilayer elastic film of claim 1, wherein the average slope of the stress-strain curve between 50% and 300%, which is measured by ASTM D-882, is the range of 0.079 to 0.236 g/cm/% (0.2 to 0.6 g/in/%).

5.  The multilayer elastic film of claim 1, wherein the multilayer elastic film comprises an $R_1/R_2$ in the range of 0.3 to 0.5, wherein $R_1$ is the unload force at 100% strain divided by the load force at 100% strain for cycle 1 and $R_2$ is the unload force at 100% strain divided by the load force at 100% strain for cycle 2, preferably the multilayer elastic film comprises an $R_1/R_2$ in the range of 0.31 to 0.45.

6.  The multilayer elastic film of claim 1, wherein the multilayer elastic film comprises an $R_1$ in the range of 0.15 to 0.35, wherein $R_1$ is the unload force at 100% strain divided by the load force at 100% strain for cycle 1.

7.  The multilayer elastic film of claim 1, wherein the multilayer elastic film comprises a tensile force less than 138 g/cm (350 g/in), an average slope of the stress-strain curve between 50% and 300% in the range of 0.079 to 0.236 g/cm/% (0.2 to 0.6 g/in/%), and an $R_1$ in the range of 0.15 to 0.35, wherein $R_1$ is the unload force at 100% strain divided by the load force at 100% strain for cycle 1.

8.  The multilayer elastic film of claim 1, wherein the multilayer elastic film has a negative hysteresis.

9.  A method of making the multilayer elastic film of claim 1 or 2 comprising:

    providing an elastomeric core layer bonded between a first layer and a second layer.

10. The method of claim 9, further comprising activating said multilayer elastic film to form an activated multilayer elastic film, wherein said activated multilayer elastic film has an LFEV in the range of 9.8 to 118 g/cm (25 to 300 g/in), wherein the LFEV is defined as follows:

$$LFEV = (T_{300}) \times (R_1/R_2) \times (T_{300}/T_{50}),$$

wherein $T_{300}$ represents the tensile force in the machine direction in 300% strain as measured in g/cm (g/in) by ASTM D-882,
$R_1/R_2$ is calculated from a two cycle hysteresis test to 200%, wherein $R_1$ is the unload force at 100% strain divided by the load force at 100% strain for cycle 1 measured in the machine direction, and $R_2$ is the unload force at 100% strain divided by the load force at 100% strain for cycle 2 measured in the machine direction, and
$T_{300}/T_{50}$ is the ratio of the tensile force at 300% strain divided by the tensile force at 50% strain measured in the machine direction in g/cm (g/in) by ASTM D-882.

**Patentansprüche**

1. Mehrschichtige elastische Folie, umfassend:

   wenigstens eine erste und eine zweite Hautschicht, die wenigstens ein Polymer umfassen, das aus Polypropylen ausgewählt ist; und
   eine elastomere Kernschicht, die ein styrolisches Copolymer umfasst und zwischen die erste und die zweite Hautschicht geklebt ist,
   wobei die erste Hautschicht und die zweite Schicht weniger als 6% des Gesamtgewichts der mehrschichtigen elastischen Folie umfassen und wobei die mehrschichtige elastische Folie einen Niederkraftelastizitätswert (LFEV) im Bereich von 9,8 bis 118 g/cm (25 bis 300 g/Zoll) aufweist, wobei der LFEV wie folgt definiert ist:

$$LFEV = (T_{300}) \times (R_1/R_2) \times (T_{300}/T_{50}),$$

   wobei $T_{300}$ für die Zugkraft in Maschinenrichtung bei 300% Dehnung steht, gemessen in g/cm (g/Zoll) gemäß ASTM D-882,
   $R_1/R_2$ anhand eines Zwei-Zyklen-Hysteresetests bis 200% berechnet wird, wobei $R_1$ die Entlastungskraft bei 100% Dehnung dividiert durch die Belastungskraft bei 100% Dehnung für Zyklus 1, gemessen in der Maschinenrichtung, ist, und $R_2$ die Entlastungskraft bei 100% Dehnung dividiert durch die Belastungskraft bei 100% Dehnung für Zyklus 2, gemessen in der Maschinenrichtung, ist; und $T_{300}/T_{50}$ das Verhältnis der Zugkraft bei 300% Dehnung zur Zugkraft bei 50% Dehnung, gemessen in der Maschinenrichtung, in g/cm (g/Zoll) gemäß ASTM D-882 ist.

2. Mehrschichtige elastische Folie gemäß Anspruch 1, wobei die mehrschichtige elastische Folie einen LFEV im Bereich von 19,7 bis 98,4 g/cm (50 bis 250 g/Zoll), vorzugsweise im Bereich von 29,5 bis 88,6 g/cm (75 bis 225 g/Zoll), am meisten bevorzugt im Bereich von 29,5 bis 68,9 g/cm (75 bis 175 g/Zoll), aufweist.

3. Mehrschichtige elastische Folie gemäß Anspruch 1, wobei die mehrschichtige elastische Folie nicht aktiviert ist.

4. Mehrschichtige elastische Folie gemäß Anspruch 1, wobei die mittlere Steigung der Spannungs-Dehnungs-Kurve zwischen 50% und 300%, die gemäß ASTM D-882 gemessen wird, im Bereich von 0,079 bis 0,236 g/cm/% (0,2 bis 0,6 g/Zoll/%) liegt.

5. Mehrschichtige elastische Folie gemäß Anspruch 1, wobei die mehrschichtige elastische Folie ein $R_1/R_2$ im Bereich von 0,3 bis 0,5 umfasst, wobei $R_1$ die Entlastungskraft bei 100% Dehnung dividiert durch die Belastungskraft bei 100% Dehnung für Zyklus 1 ist und $R_2$ die Entlastungskraft bei 100% Dehnung dividiert durch die Belastungskraft bei 100% Dehnung für Zyklus 2 ist, wobei die mehrschichtige elastische Folie vorzugsweise ein $R_1/R_2$ im Bereich von 0,31 bis 0,45 umfasst.

6. Mehrschichtige elastische Folie gemäß Anspruch 1, wobei die mehrschichtige elastische Folie ein $R_1$ im Bereich von 0,15 bis 0,35 umfasst, wobei $R_1$ die Entlastungskraft bei 100% Dehnung dividiert durch die Belastungskraft bei 100% Dehnung für Zyklus 1 ist.

**7.** Mehrschichtige elastische Folie gemäß Anspruch 1, wobei die mehrschichtige elastische Folie eine Zugkraft von weniger als 138 g/cm (350 g/Zoll), eine mittlere Steigung der Spannungs-Dehnungs-Kurve zwischen 50% und 300% im Bereich von 0,079 bis 0,236 g/cm/% (0,2 bis 0,6 g/Zoll/%) und ein $R_1$ im Bereich von 0,15 bis 0,35 umfasst, wobei $R_1$ die Entlastungskraft bei 100% Dehnung dividiert durch die Belastungskraft bei 100% Dehnung für Zyklus 1 ist.

**8.** Mehrschichtige elastische Folie gemäß Anspruch 1, wobei die mehrschichtige elastische Folie eine negative Hysterese aufweist.

**9.** Verfahren zur Herstellung der mehrschichtigen elastischen Folie gemäß Anspruch 1 oder 2, umfassend:

Bereitstellen einer elastomeren Kernschicht, die zwischen eine erste Schicht und eine zweite Schicht geklebt ist.

**10.** Verfahren gemäß Anspruch 9, das weiterhin das Aktivieren der mehrschichtigen elastischen Folie unter Bildung einer aktivierten mehrschichtigen elastischen Folie umfasst, wobei die aktivierte mehrschichtige elastische Folie einen LFEV im Bereich von 9,8 bis 118 g/cm (25 bis 300 g/Zoll) aufweist, wobei der LFEV wie folgt definiert ist:

$$LFEV = (T_{300}) \times (R_1/R_2) \times (T_{300}/T_{50}),$$

wobei $T_{300}$ für die Zugkraft in Maschinenrichtung bei 300% Dehnung steht, gemessen in g/cm (g/Zoll) gemäß ASTM D-882,

$R_1/R_2$ anhand eines Zwei-Zyklen-Hysteresetests bis 200% berechnet wird, wobei $R_1$ die Entlastungskraft bei 100% Dehnung dividiert durch die Belastungskraft bei 100% Dehnung für Zyklus 1, gemessen in der Maschinenrichtung, ist, und $R_2$ die Entlastungskraft bei 100% Dehnung dividiert durch die Belastungskraft bei 100% Dehnung für Zyklus 2, gemessen in der Maschinenrichtung, ist; und $T_{300}/T_{50}$ das Verhältnis der Zugkraft bei 300% Dehnung zur Zugkraft bei 50% Dehnung, gemessen in der Maschinenrichtung, in g/cm (g/Zoll) gemäß ASTM D-882 ist.

**Revendications**

**1.** Film élastique multicouches, comprenant :

au moins une première et une seconde couche de peau comprenant au moins un polymère choisi parmi les polypropylènes ; et
une couche d'âme élastomère comprenant un copolymère de styrène et liée entre lesdites première et seconde couche de peau, la première couche de peau et la seconde couche comprenant moins de 6% du poids total du film élastique multicouches, et le film élastique multicouches ayant une valeur élastique de force faible (LFEV) dans la plage de 9,8 à 118 g/cm (25 à 300 g/in), la valeur LFEV étant définie comme suit :

$$LFEV = (T_{300}) \times (R_1/R_2) \times (T_{300}/T_{50}),$$

où $T_{300}$ représente la force de traction dans le sens machine à 300% de déformation, mesurée en g/cm (g/in) par ASTM D-882,
$R_1/R_2$ est calculée à partir d'un test d'hystérésis en deux cycles jusqu'à 200%, $R_1$ étant la force de décharge à 100% de déformation divisée par la force de charge à 100% de déformation pour le cycle 1, mesurée dans le sens machine, et $R_2$ étant la force de décharge à 100% de déformation divisée par la force de charge à 100% de déformation pour le cycle 2, mesurée dans le sens machine, et
$T_{300}/T_{50}$ est le rapport de la force de traction à 300% de déformation divisée par la force de traction à 50% de déformation, mesurée dans le sens machine en g/cm (g/in) par ASTM D-882.

**2.** Film élastique multicouches selon la revendication 1, dans lequel le film élastique multicouches a une valeur LFEV dans la plage de 19,7 à 98,4 g/cm (50 à 250 g/in), de préférence dans la plage de 29,5 à 88,6 g/cm (75 à 225 g/in), plus préférablement dans la plage de 29,5 à 68,9 g/cm (75 à 175 g/in).

**3.** Film élastique multicouches selon la revendication 1, dans lequel le film élastique multicouches n'est pas activé.

4. Film élastique multicouches selon la revendication 1, dans lequel la pente moyenne de la courbe contrainte-déformation entre 50% et 300%, mesurée par ASTM D-882, se situe dans la plage de 0,079 à 0,236 g/cm/% (0,2 à 0,6 g/in/%).

5. Film élastique multicouches selon la revendication 1, dans lequel le film élastique multicouches a une valeur $R_1/R_2$ dans la plage de 0,3 à 0,5, $R_1$ étant la force de décharge à 100% de déformation divisée par la force de charge à 100% de déformation pour le cycle 1, et $R_2$ étant la force de décharge à 100% de déformation divisée par la force de charge à 100% de déformation pour le cycle 2, de préférence le film élastique multicouches a une valeur $R_1/R_2$ dans la plage de 0,31 à 0,45.

6. Film élastique multicouches selon la revendication 1, dans lequel le film élastique multicouches a une valeur $R_1$ dans la plage de 0,15 à 0,35, $R_1$ étant la force de décharge à 100% de déformation divisée par la force de charge à 100% de déformation pour le cycle 1.

7. Film élastique multicouches selon la revendication 1, dans lequel le film élastique multicouches a une force de traction inférieure à 138 g/cm (350 g/in), une pente moyenne de la courbe contrainte-déformation entre 50% et 300% dans la plage de 0,079 à 0,236 g/cm/% (0,2 à 0,6 g/in/%) et une valeur $R_1$ dans la plage de 0,15 à 0,35, $R_1$ étant la force de décharge à 100% de déformation divisée par la force de charge à 100% de déformation pour le cycle 1.

8. Film élastique multicouches selon la revendication 1, dans lequel le film élastique multicouches a une hystérésis négative.

9. Procédé de fabrication du film élastique multicouches selon la revendication 1 ou 2, comprenant l'étape consistant à :

   prévoir une couche d'âme élastomère liée entre une première couche et une seconde couche.

10. Procédé selon la revendication 9, consistant, en outre, à activer ledit film élastique multicouches pour former un film élastique multicouches activé, ledit film élastique multicouches activé ayant une valeur LFEV dans la plage de 9,8 à 118 g/cm (25 à 300 g/in), la valeur LFEV étant définie comme suit :

$$LFEV = (T_{300}) \times (R_1/R_2) \times (T_{300}/T_{50}),$$

où $T_{300}$ représente la force de traction dans le sens machine à 300% de déformation, mesurée en g/cm (g/in) par ASTM D-882,
$R_1/R_2$ est calculée à partir d'un test d'hystérésis en deux cycles jusqu'à 200%, $R_1$ étant la force de décharge à 100% de déformation divisée par la force de charge à 100% de déformation pour le cycle 1, mesurée dans le sens machine, et $R_2$ étant la force de décharge à 100% de déformation divisée par la force de charge à 100% de déformation pour le cycle 2, mesurée dans le sens machine, et
$T_{300}/T_{50}$ est le rapport de la force de traction à 300% de déformation divisée par la force de traction à 50% de déformation, mesurée dans le sens machine en g/cm (g/in) par ASTM D-882.

Fig. 1

Fig 2.

## REFERENCES CITED IN THE DESCRIPTION

**Patent documents cited in the description**

- WO 016065 A
- US 6472084 B, Middlesworth
- US 5733628 A, Pelkie